# EUROPÄISCHE PATENTSCHRIFT

(11) **EP 2 931 211 B1**
(45) Veröffentlichungstag und Bekanntmachung des Hinweises auf die Patenterteilung: **11.07.2018**
(21) Anmeldenummer: 13792392.6
(22) Anmeldetag: 20.11.2013
(51) Int. Cl.: A61K 8/19, A61K 8/22, A61Q 5/08, A61Q 5/10, A61K 8/60

(54) **REDUZIERUNG DES AMMONIAKGERUCHS IN MITTELN ZUM OXIDATIVEN FÄRBEN UND/ODER AUFHELLEN VON KERATINISCHEN FASERN**
REDUCTION OF AMMONIA ODOR IN AGENTS FOR THE OXIDATIVE DYEING AND/OR BLEACHING OF KERATIN FIBERS
RÉDUCTION DE L'ODEUR D'AMMONIAC DANS DES AGENTS DESTINÉS À COLORER ET/OU ECLAIRCIR DES FIBRES KÉRATINIQUES

(30) Priorität: 14.12.2012 DE 102012223204
(43) Veröffentlichungstag der Anmeldung: 21.10.2015
(73) Patentinhaber: Henkel AG & Co. KGaA, 40589 Düsseldorf (DE)
(72) Erfinder: NEUBA, Constanze, 41516 Grevenbroich (DE); JANßEN, Frank, 51103 Köln (DE)
(86) Internationale Anmeldenummer: PCT/EP2013/074219
(87) Internationale Veröffentlichungsnummer: WO 2014/090525

(56) Entgegenhaltungen:
- EP-A1- 0 370 273
- WO-A2-2004/000248
- DE-A1-102005 005 199
- JP-A- 2007 191 459
- JP-A- 2011 037 750
- US-A- 5 716 418
- US-A1- 2010 154 143
- Anonymous: "Cocochem.ph : Fatty Alcohol and Fatty Alcohol Flakes", , 31 December 2000 (2000-12-31), XP055296134, Retrieved from the Internet: URL:http://www.cocochem.ph/alcohol.html [retrieved on 2016-08-18]

## Beschreibung

Gegenstand der vorliegenden Erfindung sind Mittel zum oxidativen Färben und/oder Aufhellen von keratinischen Fasern, insbesondere menschlichen Haaren, enthaltend in einem kosmetischen Träger eine spezielle Kombination aus zwei Alkylglucosiden mit verschiedenen Alkyl-Kettenlängen, Ammoniak und mindestens einem Oxidationsmittel. Weiterhin betrifft die Erfindung die Verwendung dieser speziellen Kombination zur Reduktion des Ammoniak-Geruchs vor, während und nach dem Färbe- und/oder Aufhellvorgang.

Zur Bereitstellung farbverändernder kosmetischer Mittel, insbesondere für keratinische Fasern, wie beispielsweise Haare, kennt der Fachmann je nach Anforderung an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Die Oxidationsfärbemittel zeichnen sich durch hervorragende, lang anhaltende Färbeergebnisse aus. Neben der Färbung ist auch das Aufhellen bzw. Blondieren der eigenen Haarfarbe der ganz spezielle Wunsch vieler Verbraucher. Dazu werden die die Faser färbenden natürlichen oder künstlichen Farbstoffe meist oxidativ unter Einsatz von entsprechenden Oxidationsmitteln, wie beispielsweise Wasserstoffperoxid, entfärbt.

Um eine zufrieden stellende Färbe- und Aufhellleistung zu entfalten, benötigen oxidative Färbe- bzw. Aufhellmittel bei der Anwendung in der Regel einen alkalischen pH-Wert, insbesondere bei pH-Werten zwischen 8,5 und 10,5 werden optimale Ergebnisse erzielt.

Zur Einstellung dieser pH-Werte ist bis zum heutigen Zeitpunkt Ammoniak das Alkalisierungsmittel der Wahl. Mit Ammoniak lässt sich nicht nur der für die Farbstoffbildung notwendige pH-Bereich einstellen, sondern Ammoniak sorgt auch in stärkerem Maß als alle anderen bekannten Alkalisierungsmittel für eine Quellung der Haare. Gleichzeitig wirkt Ammoniak - ebenfalls in stärkerem Ausmaß als alle anderen handelsüblichen Alkalisierungsmittel - als Penetrations- bzw. Penetrationshilfsmittel.

Aus diesen Gründen werden bei Einsatz von Ammoniak in oxidativen Färbemitteln im Vergleich zu anderen Alkalisierungsmitteln (wie beispielsweise Kalium- oder Natriumhydroxid, Alkanolaminen oder Carbonaten wie Natriumcarbonat oder Kaliumcarbonat) intensivere Färbungen und signifikant bessere Grauabdeckungen erhalten.

Bedingt durch die von Anfang an höheren Farbintensitäten sind auch die Echtheitseigenschaften der mit Hilfe von Ammoniak erzeugten Haarfärbungen besser. Insbesondere besitzen gefärbte Haare dann die besten Waschechtheiten, wenn Ammoniak als Alkalisierungsmittel gewählt wurde.

Die mit dem Einsatz von Ammoniak verbundenen anwendungstechnischen Vorteile sind so vielfältig, dass Ammoniak trotz seines unangenehmen, stechenden Geruchs in einer Vielzahl handelsüblicher oxidativer Färbe- und Aufhellmittel verwendet wird.

Aus der Literatur sind bereits umfangreiche Bestrebungen zur Reduzierung des Ammoniak-Geruchs bekannt. Hierbei bestehen zur Minimierung des Geruchs drei prinzipielle Möglichkeiten: Als erste Möglichkeit nennt die Literatur die Variation des Alkalisierungsmittels und damit den teilweisen oder vollständigen Ersatz von Ammoniak durch geruchslose Alternativen.

So existiert beispielsweise eine Vielzahl von Rezepturen, welche ein Gemisch von Ammoniak und Monoethanolamin oder ausschließlich Monoethanolamin als Alkalisierungsmittel einsetzen. Der vollständige oder teilweise Ersatz von Ammoniak hat jedoch generell eine schlechtere Penetration der Farbstoffe in das Haar hinein zur Folge, was sich wie zuvor beschrieben insbesondere in schlechteren Grauabdeckungen und einer schlechteren Waschechtheiten niederschlägt. Wenn die Entwicklung von besonders haltbaren Nuancen im Fokus steht, ist der Einsatz von Monoethanolamin deshalb keine Option.

Die WO 2006060570 und WO 2006060565 schlagen für die Bereitstellung von oxidativen Färbemitteln mit geringer Geruchsbelastung den Einsatz von Carbonaten oder Carbonatquellen als Alkalisierungsmittel vor. Es ist jedoch ebenfalls literaturbekannt, dass Carbonate in Kombination mit Oxidationsmitteln die Haare in stärkerem Ausmaß schädigen können. Die durch die Carbonate hervorgerufene zusätzliche Schädigung des Haares mag bei Anwendung des Färbemittels auf unbehandeltem bzw. ungeschädigtem Haar wenig problematisch sein, kann sich jedoch bei Personen, die ihr Haar regelmäßig färben bzw. blondieren, zu schweren, kumulativen Schäden aufsummieren. Wird eine stärkere Aufhellung und/oder regelmäßige Färbung gewünscht, stellt der Einsatz von Carbonaten daher ebenfalls keine gangbare Alternative dar.

Eine zweite prinzipielle Möglichkeit zur Reduktion des Ammoniak-Geruchs besteht im Zusatz von speziellen Parfumstoffen, welche den Ammoniak-Geruch überdecken sollen. Dieser Weg wird beispielsweise in der WO 2005/110499 beschritten. Parfumstoffe können jedoch unter den alkalischen Lagerbedingungen instabil sein, so dass die Gefahr besteht, dass die Duftstoffe während der Lagerung abgebaut oder strukturell verändert werden, was sich auch in einer unvorhersehbaren Änderung des Geruchs niederschlägt. Da entsprechende Veränderungen oftmals erst nach mehreren Monaten oder sogar Jahren wahrnehmbar werden, wird der Einsatz von neuen bzw. unbekannten Parfums als problematisch eingestuft.

Eine dritte prinzipielle Möglichkeit zur Reduktion des Ammoniakgeruchs besteht in einer Optimierung der Formulierung. Hierbei gilt es, die Träger-Bestandteile der Formulierung so auszuwählen, dass diese einen optimalen Rückhalt des Ammoniaks in der Formulierung gewährleisten und auf diese Weise seinen Geruch minimieren. Es ist jedoch ebenfalls bekannt, dass die Formulierung, die in ihr enthaltenen Fettstoffe, ihre Emulgatoren, Tenside und ihre Viskosität wesentlichen Einfluss auf die Färbeleistung nehmen. Bei der Modifikation der Formulierung muß eine Verschlechterung der Färbeleistung daher auf jeden Fall vermieden werden.

Beispielsweise schlägt die JP 2007191459 zur Reduktion des Ammoniak-Geruchs in Haarfärbemitteln den Einsatz von kationischen Tensiden, Phophateestern und aliphatischen Alkoholen vor.

Die JP 2003040750 offenbart, dass der Ammoniak-Geruch in Blondiermittel dann besonders gering ist, wenn den Mitteln mindestens 5 % einer kristallinen Komponente zugesetzt wird.

In US 5716418 werden Haarfärbezusammensetzungen beschrieben, welche in einem wässrigen Träger Alkylglycoside und Oxidationsfarbstoffvorprodukte in bestimmten Mengenbereichen enthalten. Die Alkylketten der Alkylglycoside umfassen hierbei 12 bis 16 C-Atome.

JP 2011037750 beschreibt schaumförmige Haarfärbemittel in non-Aerosol Form, welche neben C6-C22 Alkylglucosiden auch Ammoniak sowie Oxidationsfarbstoffvorprodukte vom Entwickler- und vom Kupplertyp enthalten.

US 2010/154143 wird ein Haarfärbeverfahren beansprucht, bei welchem Haare mit einer Zubereitung enthaltend o-Diphenole, Metallsalze, Wasserstoffperoxid, Carbonate und Tenside behandelt werden. Bei den Tensiden kann es sich auch um Alkylpolyglycoside handeln.

WO 2004/000248 beschreibt kosmetische Zusammensetzungen, enthaltend die Kombination aus speziellen vernetzten Copolymeren mit einem weiteren Wirkstoff. Bei dem weiteren Wirkstoff handelt es sich unter anderem um C8-C22-Alkylglycoside.

DE 102005005199 schließlich beschreibt die Verwendung von Alkylglucosiden zur Verbesserung der mikrobiologischen Stabilität von kosmetischen Zubereitungen.

Insbesondere die dauerhafte Geruchsminimierung über die gesamte Anwendungsdauer ist nur sehr schwierig zu erreichen. Die Zeitspanne, innerhalb der sich der Anwender von Haarfarben in Kontakt mit dem Färbemittel befindet, reicht von der Herstellung der Anwendungsmischung über deren Auftragung auf die Haare und den Zeitraum des Einwirkens bis zum Auswaschen der Formulierung. Bei üblichen Einwirkzeiten von 30 bis 45 Minuten kann der gesamte Prozess bis zu 90 Minuten, im Höchstfall bis zu zwei Stunden Zeit in Anspruch nehmen. Eine geruchliche Abdeckung des Ammoniaks, die über diesen gesamten Zeitraum wirksam ist, stellt die höchste Herausforderung dar. Gerade auf diesem Feld besteht noch starker Optimierungsbedarf, und eine optimale Möglichkeit zur dauerhaften Reduktion des Ammoniak-Geruchs ist aus dem Stand der Technik bislang nicht bekannt.

Aufgabe der vorliegenden Erfindung war daher die Bereitstellung von Mitteln zum oxidativen Färben und/oder Aufhellen von Haaren mit reduziertem Ammoniakgeruch. Hierbei sollen die Mittel keine Einbußen in ihrer färberischen Leistung, insbesondere bei ihrer Grauabdeckung und ihrer Waschechtheit, aufweisen. Darüber hinaus soll die Anwendung der Mittel nicht mit einer höheren Haarschädigung verbunden sein.

Es war hierbei insbesondere die Aufgabe der vorliegenden Erfindung, über die gesamte Anwendungsdauer eine Reduktion des Ammoniakgeruchs zu erzielen. Auch am Ende der Anwendung sollte die geruchliche Wahrnehmung des Ammoniaks immer noch wirksam minimiert sein.

Überraschenderweise hat sich im Zuge der zu dieser Erfindung führenden Arbeiten herausgestellt, dass es möglich ist, die geruchliche Wahrnehmung von Ammoniak in Mitteln zum Färben und/oder Aufhellen von keratinischen Fasern wirksam über den gesamten Anwendungszeitraum zu minimieren, wenn den Mitteln neben einem Oxidationsmittel und Ammoniak eine Kombination aus zwei verschiedenen Alkylglucosiden mit unterschiedlicher Alkylkettenlänge zugesetzt wird.

Ein erster Gegenstand der vorliegenden Erfindung ist daher ein Mittel zum oxidativen Färben und/oder Aufhellen von keratinischen Fasern, insbesondere menschlichen Haaren, enthaltend in einem kosmetischen Träger - bezogen auf das Gesamtgewicht des anwendungsbereiten Mittels -
(a) ein oder mehrere Alkylglucoside der Formel (I) in einer Gesamtmenge von 0,9 bis 1,5 Gew.-% 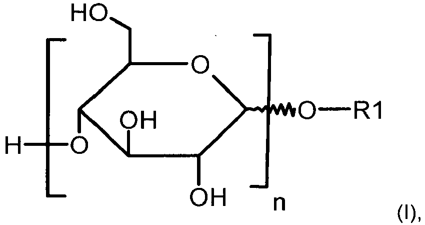 worin R1 für eine unverzweigte oder verzweigte, gesättigte oder ungesättigte C₂₀-C₂₈-Alkylgruppe steht und
   n für eine ganze Zahl von 1 bis 10 steht,
(b) ein oder mehrere Alkylglucoside der Formel (II) in einer Gesamtmenge von 1,2 bis 8,0 Gew.-%, 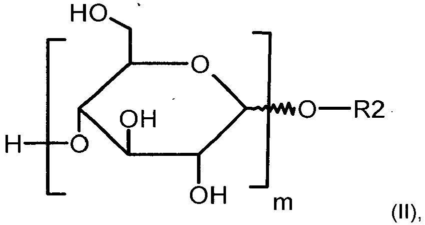 worin R2 für eine unverzweigte oder verzweigte, gesättigte oder ungesättigte C₈-C₁₈-Alkylgruppe steht und
   m für eine ganze Zahl von 1 bis 10 steht,
(c) 0,3 bis 5,5 Gew.-% Ammoniak und
(d) mindestens ein Oxidationsmittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Unter dem erfindungsgemäß verwendeten Begriff "Mittel zum Färben und/oder Aufhellen" von Keratinfasern werden oxidative Färbemittel und oxidative Aufhellmittel (Blondiermittel) verstanden. Oxidative Färbemittel enthalten Oxidationsfarbstoffvorprodukte, sogenannte Entwickler und Kupplerkomponenten. Entwickler und Kuppler diffundieren getrennt in die Keratinfaser hinein und bilden unter dem Einfluss von Ammoniak als Alkalisierungsmittel und einem Oxidationsmittel (meist Wasserstoffperoxid) in chemischer Reaktion miteinander die eigentlichen Farbstoffe aus. Abhängig von der Menge des eingesetzten Oxidationsmittels wird die Keratinfaser während der Färbung gleichzeitig mehr oder weniger stark aufgehellt, da das Oxidationsmittel nicht nur den Farbstoffbildungsprozess von Entwicklern und Kupplern initiiert, sondern auch die haareigenen Pigmente (Melanine) oxidativ zerstört.

Je nach Einsatzmengen der Oxidationsfarbstoffvorprodukte und des Oxidationsmittel kann es sich bei der oxidativen Färbung daher vornehmlich um eine Färbung (mit hohem Farbstoffanteil) oder vornehmlich um eine Aufhellung (mit hohem Anteil an Oxidationsmittel) handeln. In letzterem Fall werden die Oxidationsfarbstoffvorprodukte hauptsächlich zur Nuancierung des Aufhellergebnisses eingesetzt. Oxidative Blondiermittel können zur Nuancierung ebenfalls Oxidationsfarbstoffvorprodukte enthalten, können jedoch, wenn eine reine Aufhellung ohne weitere Nuancierung gewünscht wird, ebenso auch frei von Oxidationsfarbstoffvorprodukten sein. Zur moderaten Aufhellung wird in oxidativen Blondiermitteln als Oxidationsmittel oft Wasserstoffperoxid (typischerweise in Form seiner wässrigen Lösung) allein eingesetzt. Wird eine stärkere Aufhellleistung gewünscht, kann auch Wasserstoffperoxid in Kombination mit weiteren stärkeren Oxidationsmitteln wie Persulfatsalzen (Ammoniumperoxodisulfat, Natriumperoxodisulfat und/oder Kaliumperoxodisulfat) verwendet werden. Die erfindungsgemäßen Mittel enthalten die erfindungswesentlichen Bestandteile in einem kosmetischen Träger, bevorzugt in einem geeigneten wässrigen, alkoholischen oder wässrigalkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Als ersten wesentlichen Formulierungsbestandteil enthalten die erfindungsgemäßen Mittel - bezogen auf das Gesamtgewicht des anwendungsbereiten Mittels - ein oder mehrere Alkylglucoside (a) der Formel (I) in einer Gesamtmenge von 0,9 bis 1,5 Gew.-% 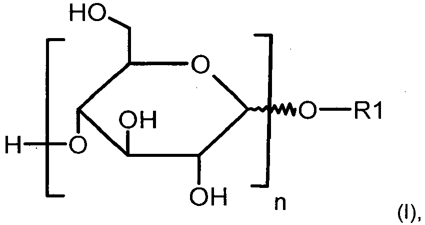 worin R1 für eine unverzweigte oder verzweigte, gesättigte oder ungesättigte C₂₀-C₂₈-Alkylgruppe steht und n für eine ganze Zahl von 1 bis 10 steht.

Unter einer unverzweigten Alkylgruppe wird eine lineare Alkylgruppe verstanden.

Unter Alkylglucosiden werden im Sinne der vorliegenden Erfindung die Glycoside von Glucose verstanden, wobei die Ausbildung der glycosidischen Bindung in Form einer Kondensationsreaktion ausgehend von der anomeren Hydroxygruppe des Glucoserestes (vorliegend in seiner Pyranoseform) mit der alkoholischen OH-Gruppe eines C₂₀-C₂₈-Hydroxyalkylrestes erfolgt.

Die Ausbildung der glycosidischen Funktionalität erfolgt ausgehend von Glucose in ihrer Pyranoseform, wobei sowohl die Glucoside ausgehend von α-Glucose (Formel Ia) als auch von der β-Glucose (Formel Ib) von der Erfindung mit umfasst sind.

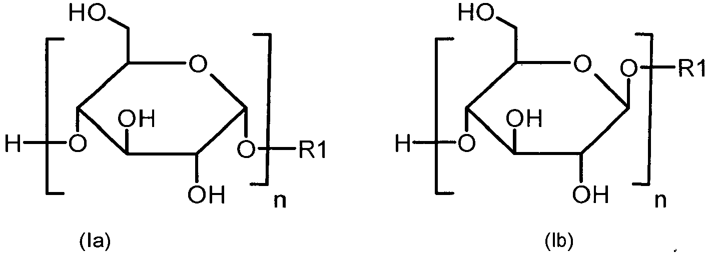

Der Rest R1 steht für eine unverzweigte oder verzweigte, gesättigte oder ungesättigte C₂₀-C₂₈-Alkylgruppe, und n steht für eine ganze Zahl von 1 bis 10.

Es hat sich herausgestellt, dass der Ammoniak-Geruch besonders dann effektiv minimiert wird, wenn der Rest R1 für eine unverzweigte, gesättigte C₂₀-C₂₄-Alkylgruppe, bevorzugt für eine unverzweigte, gesättigte C₂₀-C₂₂-Alkylgruppe und ganz besonders bevorzugt für eine unverzweigte, gesättigte C₂₀-Alkylgruppe steht.

Weiterhin wird die erfindungsgemäße Aufgabenstellung insbesondere dann optimal gelöst, wenn n für eine Zahl von 1 bis 4, bevorzugt für die Zahlen 1 und 2, und ganz besonders bevorzugt für die Zahl 1 steht.

Ganz besonders bevorzugt wird als Alkylglucosid der Formel (I) die Verbindung (Ic) eingesetzt.

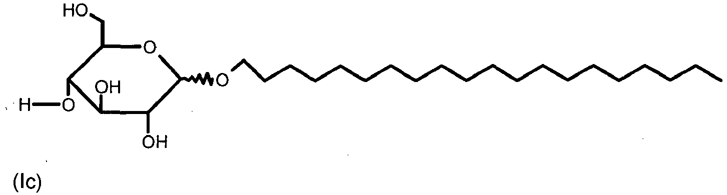

In einer besonders bevorzugten Ausführungsform ist ein Mittel zum Färben und/oder Aufhellen von keratinischen Fasern deshalb dadurch gekennzeichnet, dass es als erstes Alkylglucosid (a) mindestens eine Verbindung der Formel (I) enthält, bei welcher
R1 für eine unverzweigte, gesättigte C₂₀-Alkylgruppe steht und n für die Zahl 1 steht.

Ein entsprechendes besonders bevorzugtes Alkylglucosid (a) mit R1 gleich einer C₂₀-Alkylgruppe ist beispielsweise unter dem Handelsnamen Montanov 202 bekannt.

Effektiv ist die Minimierung des Ammoniakgeruchs insbesondere auch dann, wenn das oder die Alkylglucoside (a) der Formel (I) in bestimmten Mengenbereichen eingesetzt werden. Das oder die Alkylglucoside der Formel (I) werden in einer Gesamtmenge von 0,9 bis 1,5 Gew.-% - jeweils bezogen auf das Gesamtgewicht des anwendungsbereiten Mittels - eingesetzt.

Ein erfindungsgemäßes Mittel ist daher dadurch gekennzeichnet, dass es ein oder mehrere Alkylglucoside (a) der Formel (I) in einer Gesamtmenge von 0,9 bis 1,5 Gew.-% - bezogen auf das Gesamtgewicht des anwendungsbereiten Mittels - enthält.

Als zweiten wesentlichen Formulierungsbestandteil enthalten die erfindungsgemäßen Mittel ein oder mehrere Alkylglucoside (b) der Formel (II) in einer Gesamtmenge von 1,2 bis 8,0 Gew.-%, 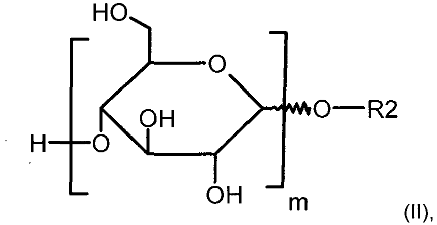 worin R2 für eine unverzweigte oder verzweigte, gesättigte oder ungesättigte C₈-C₁₈-Alkylgruppe steht und m für eine ganze Zahl von 1 bis 10 steht.

Auch die Ausbildung von Glucosiden der Formel (II) erfolgt ausgehend von Glucose in ihrer Pyranoseform, wobei auch hier die Glucoside ausgehend von α-Glucose (Formel IIa) als auch von der β-Glucose (Formel IIb) von der Erfindung mit umfasst sind.

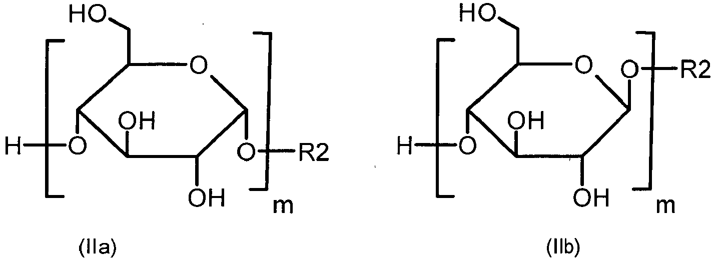

Wenn ausgewählte Vertreter von Alkylglucosiden der Formel (II) zusammen mit den weiteren wesentlichen Bestandteilen (a), (c) und (d) im erfindungsgemäßen Mittel eingesetzt werden, lässt sich der Geruch des Ammoniaks besonders gut, effektiv und über einen langen Zeitraum unterdrücken.

Es ist daher bevorzugt wenn der Rest R2 für eine unverzweigte, gesättigte C₁₂-C₁₈-Alkylgruppe steht. Weiter bevorzugt steht der Rest R2 für eine lineare, gesättigte C₁₄-C₁₆-Alkylgruppe, und ganz besonders bevorzugt steht der Rest R2 für eine lineare, gesättigte C₁₆-C₁₈-Alkylgruppe.

Weiterhin ist es im Hinblick auf die geruchliche Wahrnehmung des Ammoniaks vorteilhaft, wenn m für eine ganze Zahl von 1 bis 4 steht. Weiter bevorzugt steht m die Zahlen 1 oder 2, und explizit ganz besonders bevorzugt steht m für die Zahl 1.

Ganz besonders bevorzugt wird als Alkylglucosid der Formel (II) mindestens eine Verbindung ausgewählt aus den Formeln (IIc) und (IId) eingesetzt.

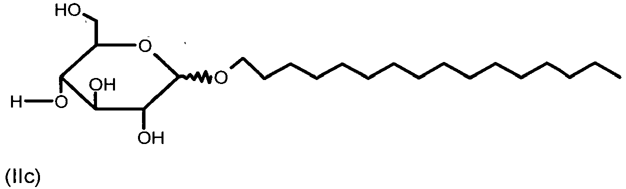

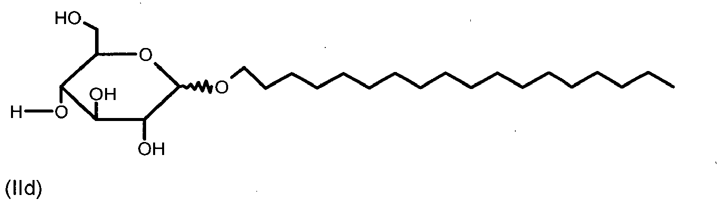

In einer weiteren besonders bevorzugten Ausführungsform ist ein erfindungsgemäßes Mittel dadurch gekennzeichnet, dass es als zweites Alkylglucosid (b) mindestens eine Verbindung der Formel (II) enthält, bei welcher
R2 für eine unverzweigte, gesättigte C₁₆-Alkylgruppe oder eine unverzweigte, gesättigte C₁₈-Alkylgruppe steht und
m für die Zahl 1 steht.

Ein besonders bevorzugtes Alkylglucosid (b) mit R2 gleich einer C₁₆-C₁₈-Alkylgruppe ist beispielsweise unter dem Handelsnamen Montanov 68 bekannt.

Im Hinblick auf eine optimale Lösung der erfindungsgemäßen Aufgabenstellung sind auch das bzw. die Alkylglucoside der Formel (II) bevorzugt in bestimmten Mengenbereichen im erfindungsgemäßen Mittel enthalten. Das erfindungsgemäße Mittel enthält ein oder mehrere Alkylglucoside (b) der Formel (II) in einer Gesamtmenge von 1,2 bis 8,0 Gew.-%, bevorzugt von 1,6 bis 6,5 Gew.-%, weiter bevorzugt von 2,0 bis 5,0 Gew.-% und besonders bevorzugt von 2,4 bis 3,5 Gew.-% - bezogen auf das Gesamtgewicht des anwendungsbereiten Mittels.

Daher ist ein erfindungsgemäßes Mittel dadurch gekennzeichnet, dass es ein oder mehrere Alkylglucoside (b) der Formel (II) in einer Gesamtmenge von 1,2 bis 8,0 Gew.-%, bevorzugt von 1,6 bis 6,5 Gew.-%, weiter bevorzugt von 2,0 bis 5,0 Gew.-% und besonders bevorzugt von 2,4 bis 3,5 Gew.-% - bezogen auf das Gesamtgewicht des anwendungsbereiten Mittels - enthält.

Darüber hinaus hat sich herausgestellt, dass es im Hinblick auf eine möglichst lange anhaltende geruchliche Abdeckung des Ammoniaks von Vorteil ist, wenn die Alkylglucoside der Formel (I) und die Alkylglucoside der Formel (II) in bestimmten Mengenverhältnissen zueinander eingesetzt werden.

In diesem Zusammenhang ist es insbesondere von Vorteil, wenn die Gesamtmenge der im anwendungsbereiten Mittel eingesetzten Alkylglucoside der Formel (II) mindestens doppelt so hoch ist wie die Gesamtmenge der im anwendungsbereiten Mittel enthaltenen Alkylglucoside der Formel (I). In einer weiteren ganz besonders bevorzugten Ausführungsform ist ein erfindungsgemäßes Mittel daher dadurch gekennzeichnet, dass das Mengenverhältnis aller im anwendungsbereiten Mittel enthaltenen Alkylglucoside der Formel (I) zu allen im anwendungsbereiten Mittel enthaltenen Alkylglucosiden der Formel (II) bei 1: 2 bis 1:10, bevorzugt bei 1:2 bis 1:5, liegt.

Als erfindungswesentliches Alkalisierungsmittel und als dritte Komponente (c) enthalten die erfindungsgemäßen Mittel weiterhin 0,3 bis 5,5 Gew.-% Ammoniak.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

Es hat sich herausgestellt, dass die geruchliche Wahrnehmung des Ammoniaks besonders effektiv und über einen besonders langen Zeitraum minimiert werden kann, wenn Ammoniak (c) und die weiteren wesentlichen Formulierungsbestandteile (a) und (b) in einem speziellen Mengenverhältnis zueinander stehen. Entsprechend ist es besonders bevorzugt, wenn Ammoniak in dem erfindungsgemäßen Mittel in einem bestimmten Mengenbereich eingesetzt wird.

Ein Mittel zum Färben und/oder Aufhellen von keratinischen Fasern ist daher dadurch gekennzeichnet, dass es Ammoniak (c) in einer Menge von 0,3 bis 5,5 Gew.-%, bevorzugt von 0,4 bis 4,5 Gew.-%, weiter bevorzugt von 0,5 bis 3,5 Gew.-% und besonders bevorzugt von 0,6 bis 1,1 Gew.-% - bezogen auf das Gesamtgewicht des anwendungsbereiten Mittels - enthält.

Die zuvor genannten bevorzugten und besonders bevorzugten Mengenangaben an Ammoniak (c) gehen von reinem Ammoniak als Berechnungsgrundlage aus. Wenn also ganz besonders bevorzugt 0,6 bis 1,1 Gew.-% Ammoniak (c) im anwendungsbereiten Mittel eingesetzt werden, so entspricht dies dem Einsatz einer Menge von 2,4 bis 4,4 g einer 25 Gew.-%igen Ammoniaklösung im anwendungsbereiten Färbe- und/oder Aufhellmittel.

Bei den erfindungsgemäßen Mitteln handelt es sich um Mittel zum oxidativen Färben und/oder Mittel zum oxidativen Aufhellen/Blondieren von Haaren, dementsprechend enthalten die Mittel als vierten wesentlichen Formulierungsbestandteil (d) mindestens ein Oxidationsmittel.

Üblicherweise wird als Oxidationsmittel Wasserstoffperoxid verwendet. In einer bevorzugten Ausführungsform wird das Wasserstoffperoxid als wässrige Lösung verwendet. Erfindungsgemäß bevorzugte anwendungsbereite Mittel sind dadurch gekennzeichnet, dass sie 0,5 bis 6,5 Gew.-%, bevorzugt von 1,3 bis 5,5 Gew.-%, weiter bevorzugt von 2,2 bis 5,0 Gew.-% und besonders bevorzugt von 3,5 bis 4,7 Gew.-% Wasserstoffperoxid (berechnet als 100 %-iges H₂O₂) enthalten.

In einer weiteren besonders bevorzugten Ausführungsform ist ein Mittel zum Färben und/oder Aufhellen von keratinischen Fasern daher dadurch gekennzeichnet, dass es als Oxidationsmittel (d) Wasserstoffperoxid in einer Menge von 0,5 bis 6,5 Gew.-%, bevorzugt von 1,3 bis 5,5 Gew.-%, weiter bevorzugt von 2,2 bis 5,0 Gew.-% und besonders bevorzugt von 3,5 bis 4,7 Gew.-% - bezogen auf das Gesamtgewicht des anwendungsbereiten Mittels - enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,2 bis 8,0 Gew.-%
(c) 0,3 bis 5,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,2 bis 8,0 Gew.-%
(c) 0,4 bis 4,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,2 bis 8,0 Gew.-%
(c) 0,5 bis 3,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,2 bis 8,0 Gew.-%
(c) 0,6 bis 1,1 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,6 bis 6,5 Gew.-%
(c) 0,3 bis 5,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,6 bis 6,5 Gew.-%
(c) 0,4 bis 4,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,6 bis 6,5 Gew.-%
(c) 0,5 bis 3,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 1,6 bis 6,5 Gew.-%
(c) 0,6 bis 1,1 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) 0,3 bis 5,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) 0,4 bis 4,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) 0,5 bis 3,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,0 bis 5,0 Gew.-%
(c) 0,6 bis 1,1 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) 0,3 bis 5,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) 0,4 bis 4,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) 0,5 bis 3,5 Gew.-% Ammoniak und
(d) mindestens ein Oxidationsmittel enthält.

Ein weiteres besonders bevorzugtes Mittel ist dadurch gekennzeichnet, dass es - bezogen auf das Gesamtgewicht des anwendungsbereiten Mittels -
(a) ein oder mehrere Alkylglucoside der Formel (I) in einer Gesamtmenge von 0,9 bis 1,5 Gew.-%
(b) ein o. mehrere Alkylglucoside der Formel (II) in einer Gesamtmenge von 2,4 bis 3,5 Gew.-%
(c) 0,6 bis 1,1 Gew.-% Ammoniak und
(d) mindestens ein Oxidationsmittel enthält.

Im Zuge der zu dieser Erfindung führenden Arbeiten hat sich herausgestellt, dass die geruchliche Abdeckung von Ammoniak weiter verbessert werden kann, wenn den erfindungsgemäßen Mittel zusätzlich ausgesuchte Fettalkohole in bestimmten Mengenbereichen beigemischt werden.

Unter Fettalkoholen sind erfindungsgemäß gesättigte oder ungesättigte, unverzweigte oder verzweigte C₈-C₂₈-Alkylgruppen mit Hydroxysubstitution zu verstehen. Ungesättigte Fettalkohole können einfach oder mehrfach ungesättigt sein. Bei einem ungesättigten Fettalkohol kann bzw. können deren C-C-Doppelbindung(en) die Cis- oder Trans-Konfiguration aufweisen.

Bevorzugte Fettalkohole sind Fettalkohole mit einer Kettenlänge von mindestens 20 C-Atomen. Besonders bevorzugt ist der Einsatz eines oder mehrerer Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol).

Die Fettalkohole der vorgenannten Gruppe werden dem erfindungsgemäßen Mittel bevorzugt in einer Gesamtmenge von 0,5 bis 7,5 Gew.-%, bevorzugt von 0,7 bis 5,0 Gew.-%, weiter bevorzugt von 0,8 bis 4,3 Gew.-% und besonders bevorzugt von 0,9 bis 2,6 Gew.-% - bezogen auf das Gesamtgewicht des anwendungsbereiten Mittels - zugesetzt.

In einer weiteren besonders bevorzugten Ausführungsform ist ein erfindungsgemäßes Mittel dementsprechend dadurch gekennzeichnet, dass es zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,5 bis 7,5 Gew.-%, bevorzugt von 0,7 bis 5,0 Gew.-%, weiter bevorzugt von 0,8 bis 4,3 Gew.-% und besonders bevorzugt von 0,9 bis 2,6 Gew.-% - bezogen auf das Gesamtgewicht des anwendungsbereiten Mittels - enthält.

Zusätzlich zu den zuvor genannten Fettalkoholen können die erfindungsgemäßen Mittel auch noch weitere Fettalkoholen mit geringerer Alkylkettenlänge von 12 bis 18 C-Atomen enthalten.

Geeignete Fettalkohole aus der Gruppe von Alkoholen mit einer Kettenlänge von 12 bis 18 C-Atomen können ausgewählt werden aus der Gruppe Laurylalkohol (Dodecan-1-ol), Myristylalkohol (Tetradecan-1-ol), Cetylalkohol (Hexadecan-1-ol) und Stearylalkohol (Octadecan-1-ol).

Die Fettalkohole mit einer Kettenlänge von 12 bis 18 C-Atomen können in einer Gesamtmenge von 0,4 bis 7,5 Gew.-%, bevorzugt von 0,8 bis 6,6 Gew.-%, weiter bevorzugt von 1,2 bis 4,6 Gew.-% und besonders bevorzugt von 1,6 bis 2,6 Gew.-% - bezogen auf das Gesamtgewicht des anwendungsbereiten Mittels - in den erfindungsgemäßen Mitteln eingesetzt werden.

In einer weiteren Ausführungsform ist ein erfindungsgemäßes Mittel daher dadurch gekennzeichnet, dass es zusätzlich einen oder mehrere Fettalkohole aus der Gruppe Laurylalkohol (Dodecan-1-ol), Myristylalkohol (Tetradecan-1-ol), Cetylalkohol (Hexadecan-1-ol) und Stearylalkohol (Octadecan-1-ol) in einer Gesamtmenge von 0,4 bis 7,5 Gew.-%, bevorzugt von 0,8 bis 6,6 Gew.-%, weiter bevorzugt von 1,2 bis 4,6 Gew.-% und besonders bevorzugt von 1,6 bis 2,6 Gew.-% - bezogen auf das Gesamtgewicht des anwendungsbereiten Mittels - enthält.

Überraschenderweise hat sich herausgestellt, dass die geruchliche Abdeckung von Ammoniak jedoch wieder vermindert und damit weniger effektiv wird, wenn die Fettalkohole in zu hohen Mengenbereichen in den erfindungsgemäßen Mitteln eingesetzt werden. Im Hinblick auf die Lösung der erfindungsgemäßen Aufgabenstellung ist es daher von Vorteil, wenn die in den Mitteln enthaltenen Fettalkohole eine bestimmte Gesamtmenge nicht überschreiten.

Dementsprechend ist eine weitere bevorzugte Ausführungsform des erfindungsgemäßen Mittels dadurch gekennzeichnet, dass der Gesamtgehalt aller im anwendungsbereiten Mittel enthaltenen Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol), Brassidylalkohol ((13*E*)-Docosen-1-ol), Laurylalkohol (Dodecan-1-ol), Myristylalkohol (Tetradecan-1-ol), Cetylalkohol (Hexadecan-1-ol) und Stearylalkohol (Octadecan-1-ol) - bezogen auf das Gesamtgewicht des anwendungsbereiten Mittels - unterhalb von 8,0 Gew.-%, bevorzugt unterhalb von 7,0 Gew.-%, weiter bevorzugt unterhalb von 6,0 Gew.-% und insbesondere bevorzugt unterhalb von 5,6 Gew.-% liegt. Weiterhin ist es bevorzugt, wenn es sich bei den erfindungsgemäßen Mitteln um oxidative Färbemittel handelt. In diesem Fall enthalten die Mittel zur Ausbildung der Farbstoffe in den keratinischen Fasern zusätzlich ein oder mehrere Oxidationsfarbstoffvorprodukte.

In einer weiteren bevorzugten Ausführungsform ist ein erfindungsgemäßes Mittel dadurch gekennzeichnet, dass es zusätzlich mindestens ein Oxidationsfarbstoffvorprodukt vom Entwicklertyp und gegebenenfalls zusätzlich mindestens ein Oxidationsfarbstoffvorprodukt vom Kupplertyp enthält. Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform enthalten die erfindungsgemäßen Mittel ein oder mehrere Oxidationsfarbstoffvorprodukte in einer Gesamtmenge von 0,01 bis 4,0 Gew.-%, bevorzugt von 0,1 bis 3,5 Gew.-%, weiter bevorzugt von 0,6 bis 3,1 Gew.-% und ganz besonders bevorzugt von 1,2 bis 2,2 Gew.-%, bezogen auf das Gesamtgewicht des anwendungsbereiten Mittels.

In einer weiteren bevorzugten Ausführungsform können die erfindungsgemäßen Mittel neben Oxidationsfarbstoffvorprodukten zur weiteren Nuancierung auch mindestens einen direktziehenden Farbstoff enthalten. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17 sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)-amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Färbeergebnisse mit herausragender Farbintensität, Brillanz und guter Waschechtheit werden insbesondere dann erhalten, wenn die erfindungsgemäßen Mittel als weiteren direktziehenden Farbstoff mindestens einen Farbstoff ausgewählt aus D&C Red No. 33 (Acid Red 33), Acid Black No. 1, D&C Orange No. 4 (Acid Orange No. 4), Acid Red 18, Basic Red 76, Acid Violet 43, HC Blue No. 12, N-(2-Hydroxethyl)-4-methyl-2-nitroanilin (Methyl Yellow), HC Yellow No. 2, Red B 54 und 2-Amino-6-chlor-4-phenol enthalten.

Wie bereits zuvor beschrieben enthalten die erfindungsgemäßen Mittel als Alkalisierungs-, Quell- und Penetrationshilfsmittel Ammoniak. Der vollständige oder teilweise Austausch von Ammoniak durch alternative, geruchslose Alkalisierungsmittel kann mit verschiedensten anwendungstechnischen Nachteilen behaftet sein. Daher ist es von Vorteil, wenn die erfindungsgemäßen Mittel frei von bestimmten weiteren Alkalisierungsmitteln sind.

Das Hinzufügen von Carbonaten oder Hydrogencarboaneten ist mit einer verstärkten Haarschädigung verbunden. Daher sind bevorzugte erfindungsgemäße Mittel dadurch gekennzeichnet, dass sie keine Carbonate enthalten.

Carbonate im Sinn der vorliegenden Erfindung sind alle Salze, die als Anion Carbonat oder Hydrogencarbonat enthalten. Von dieser Definition umfasst sind anorganische Carbonatsalze wie beispielsweise Natriumcarbonat, Natriumhydrogencarbonat, Kaliumcarbonat, Kaliumhydrogen carbonat, Ammoniumcarbonat, Magnesiumcarbonat oder Calciumcarbonat. Auch die Carbonatsalze von Metallen oder Übergangsmetallen liegen innerhalb der Definition der vorliegenden Erfindung. Von der Definition der Carbonate ebenfalls mit umfasst sind die Salze von organischen Kationen (wie beispielsweise Tetraalkylammoniumionen), die als Gegenion ein Carbonat (CO₃²⁻) oder Hydrogencarbonat-Anion (HCO₃⁻) besitzen.

Definitionsgemäß enthält ein erfindungsgemäßes Mittel keine Carbonate, wenn der Gesamtgehalt an Carbonaten im erfindungsgemäßen Mittel weniger als 0,1 Gew.-%, bevorzugt weniger als 0,05 Gew.-%, besonders bevorzugt weniger als 0,01 Gew.-% und außerordentlich bevorzugt 0 Gew.-% - bezogen auf das Gesamtgewicht des anwendungsbereiten Mittels - beträgt.

Auch wenn Ammoniak vollständig oder teilweise durch Alkanolamine ersetzt wird, ist dies mit anwendungstechnischen Nachteilen verbunden. Insbesondere kann der Ersatz von Ammoniak durch Alkanolamine zu einer Verschlechterung der Waschechtheiten und der Grauabdeckungen bestimmter Nuancen führen.

Bei Alkanolaminen handelt es sich um primäre, sekundäre oder tertiäre Amine mit einem C₂-C₆-Alkylgrundkörper, der mindestens eine Hydroxylgruppe trägt. Beispielhaft als Alkanolamine können 2-Aminoethan-1-ol (Monoethanolamin), 2-Amino-2-methylpropan-1-ol, 2-Amino-2-methyl-propan-1,3-diol und Triethanolamin genannt werden.

Definitionsgemäß enthält ein erfindungsgemäßes Mittel keine Alkanolamine, wenn der Gesamtgehalt an Alkanolaminen im erfindungsgemäßen Mittel weniger als 0,1 Gew.-%, bevorzugt weniger als 0,05 Gew.-% besonders bevorzugt weniger als 0,01 Gew.-% und außerordentlich bevorzugt 0 Gew.-% - bezogen auf das Gesamtgewicht des anwendungsbereiten Mittels - beträgt.

Es ist insbesondere bevorzugt, wenn das erfindungsgemäße Mittel keine Alkanolamine der nachfolgenden Formel (III) enthält.

In einer weiteren besonders bevorzugten Ausführungsform ist ein erfindungsgemäßes Mittel dadurch gekennzeichnet, dass die Gesamtmenge aller im anwendungsbereiten Mittel enthaltenen Alkanolamine der Formel (III) - bezogen auf das Gesamtgewicht des anwendungsbereiten Mittels - weniger als 0,1 Gew.-%, bevorzugt weniger als 0,05 Gew.-%, weiter bevorzugt weniger als 0,01 Gew.-% und außerordentlich bevorzugt 0 Gew.-% beträgt, 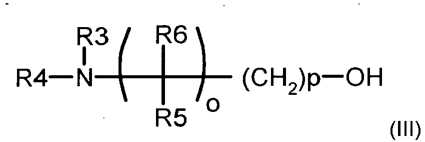 wobei
die Reste R4 und R3 unabhängig voneinander für ein Wasserstoffatom oder eine C₁-C₆-Hydroxyalkylgruppe stehen,
o für die Zahl 0 oder 1 steht,
p für eine ganze Zahl von 1 bis 6 steht, und
die Reste R5 und R6 unabhängig voneinander für eine C₁-C₆-Alkylgruppe stehen.

Im Gegensatz zu den vorgenannten Alkalisierungsmitteln hat sich der Einsatz geringer Mengen an Alkalimetallhydroxiden im Hinblick auf die geruchlichen und anwendungstechnischen Eigenschaften des Mittels nicht als nachteilig herausgestellt, daher können die Mittel Alkalimetallhydroxyide (Natriumhydroxid und/oder Kaliumhydroxid) in geringen Mengenbereichen bis zu 1 Gew.-%, bevorzugt bis zu 0,5 Gew.-%, und besonders bevorzugt bis zu 0,3 Gew.-% - bezogen auf das Gesamtgewicht des anwendungsbereiten Mittels - enthalten.

Die erfindungsgemäßen Mittel können als zusätzlichen Inhaltsstoff weiterhin mindestens einen ethoxylierten Fettalkohol mit einem Ethoxylierungsgrad von 25 bis 40 enthalten.

Unter Fettalkoholen sind wie zuvor beschrieben gesättigte oder ungesättigte, unverzweigte oder verzweigte C₈-C₂₈-Alkylgruppen mit Hydroxysubstitution zu verstehen. Ungesättigte Fettalkohole können einfach oder mehrfach ungesättigt sein. Bei einem ungesättigten Fettalkohol kann bzw. können deren C-C-Doppelbindung(en) die Cis- oder Trans-Konfiguration aufweisen.

Bevorzugte Fettalkohole als Ausgangstoffe für die Ethoxlierung sind Octan-1-ol (Octylalkohol, Caprylalkohol), Decan-1-ol (Decylalkohol, Caprinalkohol), Dodecan-1-ol (Dodecylalkohol, Laurylalkohol), Tetradecan-1-ol, (Tetradecylalkohol, Myristylalkohol), Hexadecan-1-ol (Hexadecylalkohol, Cetylalkohol, Palmitylalkohol),

Octadecan-1-ol (Octadecylalkohol, Stearylalkohol), (9*Z*)-Octadec-9-en-1-ol (Oleylalkohol), (9E)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Eicosan-1-ol (Eicosylalkohol, Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Docosan-1-ol (Docosylalkohol, Behenylalkohol), (13*E*)-Docosen-1-ol (Brassidylalkohol) und (13*Z*)-Docos-13-en-1-ol (Erucylalkohol). Innerhalb dieser Gruppe wiederum sind Hexadecan-1-ol (Hexadecylalkohol, Cetylalkohol, Palmitylalkohol) und Octadecan-1-ol (Octadecylalkohol, Stearylalkohol) ganz besonders bevorzugt Fettalkohole.

Unter Ethoxylierung (auch Oxyethylierung) wird die Reaktion der Fettalkohole mit Ethylenoxid (EO) verstanden. Durch Insertion von 25 bis 40 Gruppierungen des Typs -CH2-CH2-O- pro Fettalkohol Molekül entstehen lineare Polyether, die an einem Kettenende eine Hydroxygruppe und am anderen Kettenende die C₈-C₂₈-Alkylgruppe des Fettalkohols tragen.

Bevorzugt besitzen die Fettalkohole einen Ethoxylierungsgrad von 26 bis 38, weiter bevorzugt von 27 bis 36 und insbesondere bevorzugt von 28 bis 34.

In einer weiteren bevorzugten Ausführungsform ist ein erfindungsgemäßes Mittel dadurch gekennzeichnet, dass es zusätzlich mindestens einen ethoxylierten Fettalkohol mit einem Ethoxylierungsgrad von 25 bis 40 enthält, welcher der Formel (IV) entspricht 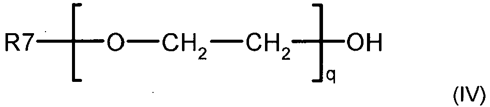 und worin
R7 für eine gesättigte oder ungesättigte, unverzweigte oder verzweigte C₈-C₂₈-Alkylgruppe, bevorzugt für eine gesättigte, unverzweigte C₁₆- oder C₁₈- Alkylgruppe, steht und
q für eine ganze Zahl von 25 bis 40, bevorzugt für eine ganze Zahl von 26 bis 38, weiter bevorzugt für eine ganze Zahl von 27 bis 36 und insbesondere bevorzugt für eine ganze Zahl von 28 bis 34 steht.

Der bzw. die ethoxylierten Fettalkohole mit einem Ethoxylierungsgrad von 25 bis 40 sind im erfindungsgemäßen Mittel in einer Gesamtmenge von 0,2 bis 1,5 Gew.-%, bevorzugt von 0,3 bis 1,2 Gew.-%, weiter bevorzugt von 0,4 bis 0,9 Gew.-% und besonders bevorzugt von 0,5 bis 0,8 Gew.-% - bezogen auf das Gesamtgewicht des anwendungsbereiten Mittels - enthalten.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten, die von den nichtionischen Tensiden der Formel (IV) verschieden sind. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus Gruppe der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten. Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit. Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 11, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in dem erfindungsgemäßen Mittel enthalten. Ferner können die erfindungsgemäßen Mittel weitere Wirk-, Hilfs- und Zusatzstoffe enthalten, wie nichtionische Polymere (beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane); Silikone, wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere); kationische Polymere wie quaternisierte Celluloseether, Polysiloxane mit quaternären Gruppen wie Dimethyldiallylammoniumchlorid-Copolymere, mit Diethylsulfat quaternierte Dimethylamino-ethylmethacrylat-Vinylpyrrolidinon-Copolymere, Vinylimidazolinium-methochlorid-Copolymere; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Die Kombinationen der zuvor beschriebenen Alkylglucoside der Formel (I) mit Alkylglucosiden der Formel (II) und gegebenenfalls ausgesuchten Fettalkoholen eignen sich hervorragend zur geruchlichen Abdeckung von Ammoniak in Mittel zum oxidativen Färben und oxidativen Aufhellen von keratinischen Fasern.

Ein weiterer Gegenstand der vorliegenden Erfindung ist daher die Verwendung der Kombination aus
(a) mindestens einem ersten Alkylglucosid der Formel (I) 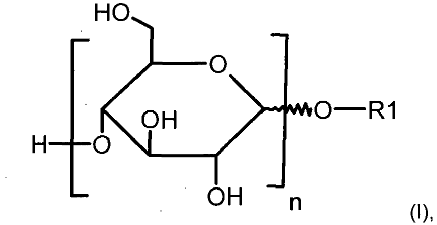 worin R1 für eine unverzweigte oder verzweigte, gesättigte oder ungesättigte C₂₀-C₂₈-Alkylgruppe steht und n für eine ganze Zahl von 1 bis 10 steht und
(b) mindestens einem zweiten Alkylglucosid der Formel (II) 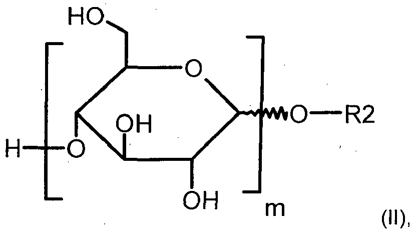 worin R2 für eine unverzweigte oder verzweigte, gesättigte oder ungesättigte C₈-C₁₈-Alkylgruppe steht und m für eine ganze Zahl von 1 bis 10 steht, und
(c) gegebenenfalls mindestens einem Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol), Brassidylalkohol ((13*E*)-Docosen-1-ol), Laurylalkohol (Dodecan-1-ol), Myristylalkohol (Tetradecan-1-ol), Cetylalkohol (Hexadecan-1-ol) und Stearylalkohol (Octadecan-1-ol) zur Reduktion des Ammoniakgeruches in Mitteln zum oxidativen Färben und/oder oxidativen Aufhellen von keratinischen Fasern, insbesondere menschlichen Haaren, wobei die Mittel in einem kosmetischen Träger - bezogen auf das Gesamtgewicht des anwendungsbereiten Mittels
   (a) ein oder mehrere Alkylglucoside der Formel (I) in einer Gesamtmenge von 0,9 bis 1,5 Gew.-% und
   (b) ein oder mehrere Alkylglucoside der Formel (II) in einer Gesamtmenge von 1,2 bis 8,0 Gew.-% und
   (c) 0,3 bis 5,5 Gew.-% Ammoniak und (d) mindestens ein Oxidationsmittel enthalten.

Bevorzugt werden erfindungsgemäßen Mittel direkt vor der Anwendung aus zwei oder mehreren getrennt verpackten Zubereitungen hergestellt. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt.

Im Falle eines oxidativen Färbemittels ist es bevorzugt, das Mittel in Form von zwei getrennt voneinander konfektionierten Komponenten bereitzustellen ("Kit of Parts"), wobei die erste Komponente (a) mindestens ein erstes Alkylglucosid der Formel (I) 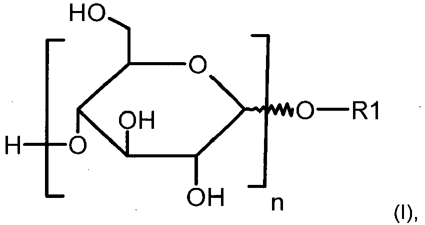 worin R1 für eine unverzweigte oder verzweigte, gesättigte oder ungesättigte C₂₀-C₂₈-Alkylgruppe steht und n für eine ganze Zahl von 1 bis 10 steht,
(b) mindestens ein zweites Alkylglucosid der Formel (II) 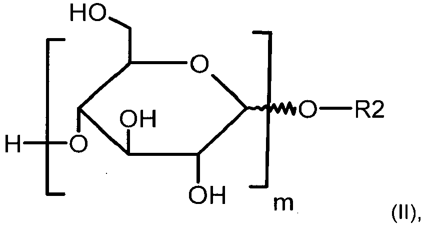 worin R2 für eine unverzweigte oder verzweigte, gesättigte oder ungesättigte C₈-C₁₈-Alkylgruppe steht und m für eine ganze Zahl von 1 bis 10 steht,
(c) Ammoniak und
   zusätzlich mindestens ein Oxidationsfarbstoffvorprodukt vom Entwicklertyp und gegebenenfallszusätzlich mindestens ein Oxidationsfarbstoffvorprodukt vom Kupplertyp enthält und wobei die zweite Komponente
(d) mindestens ein Oxidationsmittel enthält.

Vor der Anwendung werden die erste Komponente und die zweite Komponente innig miteinander vermischt, wobei das anwendungsbereite Mittel entsteht.

Bei allen Mengenangaben, die sich auf das Gesamtgewicht des anwendungsbereiten Mittels beziehen, ist ein Mittel gemeint, welches (sofern das erfindungsgemäße Mittel in Form von voneinander getrennt konfektionierten Komponenten bereitgestellt wird) durch Vermischen der verschiedenen Komponenten hergestellt und direkt zur Anwendung auf der Keratinfaser bereit ist.

Bezüglich weiterer bevorzugter Ausführungsformen der erfindungsgemäßen Kit-of-Parts und Verwendungen gilt mutatis mutandis das zu den erfindungsgemäßen Mitteln Gesagte.

### 1. Beispiele

### 1.1. Herstellung der anwendunqsbereiten Färbemittel

Es wurden die folgenden Rezepturen hergestellt. Die Mengenangaben verstehen sich, sofern nichts anderes vermerkt ist, jeweils in Gewichtsprozent.

| Formulierungsbestandteile | V1 (Gew.-%) | E1 (Gew.-%) |
|---|---|---|
| Montanov 68 (Cetearyl Alcohol, Cetearyl Glucoside) | --- | 6,00 |
| Montanov 202 (Arachidyl Alcohol, Behenyl Alcohol, Arachidyl Glucoside) | --- | 2,00 |
| Paraffinium Liquidum | 7,40 | 7,40 |
| Lanette 22 (INCI: Behenylalcohol) | 1,80 | 1,80 |
| Lanette D (INCI: Cetearylalkohol) | 1,30 | 1,30 |
| Eumulgin B 3 (INCI: Ceteareth-30) | 1,30 | 1,30 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer) | 2,00 | 2,00 |
| p-Toluylendiamin Sulfat | 1,50 | 1,50 |
| Resorcin | 0,58 | 0,58 |
| m-Aminophenol | 0,16 | 0,16 |
| 3-Amino-2-methylamino-6-methoxypyridin | 0,05 | 0,05 |
| Kaliumhydroxid (50 %ig) | 0,7 | 0,7 |
| Vitamin C | 0,05 | 0,05 |
| Hydroxyethan-1,1-diphosphonsäure 60 %ig | 0,20 | 0,20 |
| Natriumsilikat 42 (3,1 SiO₂: Na₂O) | 0,5 | 0,5 |
| Ammoniak (25 Gew.-%ige wässrige Lösung) | 5,80 | 5,80 |
| Parfum | 0,40 | 0,40 |
| Wasser | ad 100 | ad 100 |

Bei V1 handelt es sich um eine Vergleichsrezeptur, E1 ist eine erfindungsgemäße Formulierung.

Die Farbcremes wurden jeweils im Verhältnis 1:1 mit der folgenden Oxidationsmittelformierung (OX) vermischt.

| Formulierungsbestandteile | OX (Gew.-%) |
|---|---|
| Phosphorsäure 85 %ig | 0,04 |
| Wasserstoffperoxid (50 %ige, wässrige Lösung) | 12,00 |
| Emulgade F (INCI: Cetearylalcohol, PEG-40 Castor Oil, Sodium Cetearyl sulfate) | 2,10 |
| Natriumbenzoat | 0,04 |
| Dinatriumpyrophosphat | 0,30 |
| Ethylendiamintetraacetat, Dinatriumsalz | 0,15 |
| Wasser | ad 100 |

### 1.2. Bestimmung des Ammoniakgeruchs

Die zuvor hergestellten Anwendungsmischungen (V1 + OX, E1 + OX) wurden jeweils auf den Kopf eines Probanden aufgetragen. Während des Anwendungszeitraums wurde der Ammoniak-Geruch von jeweils 5 geschulten Personen zu verschiedenen Zeitpunkten (direkt nach der Anwendung nach 0 min, nach 10 min, nach 20 min und nach 30 min.) bewertet. Die Bewertung erfolgte blind, was bedeutet, dass den Personen, die die Bewertung vornahmen, nicht bekannt war, welche Rezeptur sie gerade bewerteten. Aus den Einzelbewertungen wurde jeweils der Mittelwert gebildet.

Der Ammoniak-Geruch wurde auf einer Skala von 0 (quasi kein Geruch wahrnehmbar) bis 10 (sehr starker Ammoniak-Geruch) bewertet.

**Tabelle 4: Ammoniak-Geruch während der Anwendung**

| | nach 0 min. | nach 10 min. | nach 20 min. | nach 30 min. |
|---|---|---|---|---|
| V1 + OX | 5,0 | 3,0 | 3,0 | 1,5 |
| E1 + OX | 2,25 | 2,5 | 1,5 | 1,0 |

Es zeigt sich, dass der Ammoniak-Geruch bei Anwendung der erfindungsgemäßen Formulierung sowohl direkt nach dem Auftrag der Formulierungen als auch nach einem Zeitraum von 10 Minuten, 20 Minuten und 30 Minuten als deutlich reduziert wahrgenommen wurde.

## Patentansprüche

1. Mittel zum oxidativen Färben und/oder Aufhellen von keratinischen Fasern, insbesondere menschlichen Haaren, enthaltend in einem kosmetischen Träger - bezogen auf das Gesamtgewicht des anwendungsbereiten Mittels -
(a) ein oder mehrere Alkylglucoside der Formel (I) in einer Gesamtmenge von 0,9 bis 1,5 Gew.-% 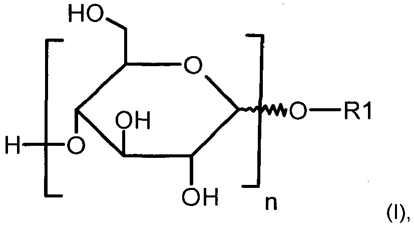 worin R1 für eine unverzweigte oder verzweigte, gesättigte oder ungesättigte C₂₀-C₂₈-Alkylgruppe steht und
n für eine ganze Zahl von 1 bis 10 steht,
(b) ein oder mehrere Alkylglucoside der Formel (II) in einer Gesamtmenge von 1,2 bis 8,0 Gew.-%, 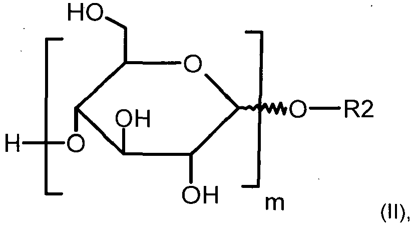 worin R2 für eine unverzweigte oder verzweigte, gesättigte oder ungesättigte C₈-C₁₈-Alkylgruppe steht und
m für eine ganze Zahl von 1 bis 10 steht,
(c) 0,3 bis 5,5 Gew.-% Ammoniak und
(d) mindestens ein Oxidationsmittel.

2. Mittel nach Anspruch 1, **dadurch gekennzeichnet, dass** es ein oder mehrere Alkylglucoside (b) der Formel (II) in einer Gesamtmenge von 1,6 bis 6,5 Gew.-%, bevorzugt von 2,0 bis 5,0 Gew.-% und besonders bevorzugt von 2,4 bis 3,5 Gew.-% - bezogen auf das Gesamtgewicht des anwendungsbereiten Mittels - enthält.

3. Mittel nach einem der Ansprüche 1 bis 2, **dadurch gekennzeichnet, dass** das Mengenverhältnis aller im anwendungsbereiten Mittel enthaltenen Alkylglucoside der Formel (I) zu allen im
anwendungsbereiten Mittel enthaltenen Alkylglucosiden der Formel (II) bei 1: 2 bis 1:10, bevorzugt bei 1:2 bis 1:5, liegt.

4. Mittel nach einem der Ansprüche 1 bis 3, **dadurch gekennzeichnet, dass** es Ammoniak (c) in einer Menge von 0,4 bis 4,5 Gew.-%, bevorzugt von 0,5 bis 3,5 Gew.-% und besonders bevorzugt von 0,6 bis 1,1 Gew.-% - bezogen auf das Gesamtgewicht des anwendungsbereiten Mittels - enthält.

5. Mittel nach einem der Ansprüche 1 bis 4, **dadurch gekennzeichnet, dass** es als Oxidationsmittel (d) Wasserstoffperoxid in einer Menge von 0,5 bis 6,5 Gew.-%, bevorzugt von 1,3 bis 5,5 Gew.-%, weiter bevorzugt von 2,2 bis 5,0 Gew.-% und besonders bevorzugt von 3,5 bis 4,7 Gew.-% - bezogen auf das Gesamtgewicht des anwendungsbereiten Mittels - enthält.

6. Mittel nach einem der Ansprüche 1 bis 5, **dadurch gekennzeichnet, dass** es zusätzlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,5 bis 7,5 Gew.-%, bevorzugt von 0,7 bis 5,0 Gew.-%, weiter bevorzugt von 0,8 bis 4,3 Gew.-% und besonders bevorzugt von 0,9 bis 2,6 Gew.-% - bezogen auf das Gesamtgewicht des anwendungsbereiten Mittels - enthält.

7. Mittel nach einem der Ansprüche 1 bis 6, **dadurch gekennzeichnet, dass** es zusätzlich einen oder mehrere Fettalkohole aus der Gruppe Laurylalkohol (Dodecan-1-ol), Myristylalkohol (Tetradecan-1-ol), Cetylalkohol (Hexadecan-1-ol) und Stearylalkohol (Octadecan-1-ol) in einer Gesamtmenge von 0,4 bis 7,5 Gew.-%, bevorzugt von 0,8 bis 6,6 Gew.-%, weiter bevorzugt von 1,2 bis 4,6 Gew.-% und besonders bevorzugt von 1,6 bis 2,6 Gew.-% - bezogen auf das Gesamtgewicht des anwendungsbereiten Mittels - enthält.

8. Mittel nach einem der Ansprüche 1 bis 7, **dadurch gekennzeichnet, dass** es zusätzlich mindestens ein Oxidationsfarbstoffvorprodukt vom Entwicklertyp und gegebenenfalls zusätzlich mindestens ein Oxidationsfarbstoffvorprodukt vom Kupplertyp enthält.

9. Mittel nach einem der Ansprüche 1 bis 8, **dadurch gekennzeichnet, dass** es als als erstes Alkylglucosid (a) mindestens eine Verbindung der Formel (I) enthält, bei welcher R1 für eine unverzweigte, gesättigte C₂₀-Alkylgruppe steht und n für die Zahl 1 steht.

10. Mittel nach einem der Ansprüche 1 bis 9, **dadurch gekennzeichnet, dass** es als zweites Alkylglucosid (b) mindestens eine Verbindung der Formel (II) enthält, bei welcher
R2 für eine unverzweigte, gesättigte C₁₆-Alkylgruppe oder eine unverzweigte, gesättigte C₁₈-Alkylgruppe steht und m für die Zahl 1 steht.

11. Mittel nach einem der Ansprüche 1 bis 10, **dadurch gekennzeichnet, dass** der Gesamtgehalt aller im anwendungsbereiten Mittel enthaltenen Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol), Brassidylalkohol ((13*E*)-Docosen-1-ol), Laurylalkohol (Dodecan-1-ol), Myristylalkohol (Tetradecan-1-ol), Cetylalkohol (Hexadecan-1-ol) und Stearylalkohol (Octadecan-1-ol) - bezogen auf das Gesamtgewicht des anwendungsbereiten Mittels - unterhalb von 8,0 Gew.-%, bevorzugt unterhalb von 7,0 Gew.-%, weiter bevorzugt unterhalb von 6,0 Gew.-% und insbesondere bevorzugt unterhalb von 5,6 Gew.-% liegt.

12. Mittel nach einem der Ansprüche 1 bis 11, **dadurch gekennzeichnet, dass** die Gesamtmenge aller im anwendungsbereiten Mittel enthaltenen Alkanolamine der Formel (III) - bezogen auf das Gesamtgewicht des anwendungsbereiten Mittels - weniger als 0,1 Gew.-%, bevorzugt weniger als 0,05 Gew.-%, weiter bevorzugt weniger als 0,01 Gew.-% und außerordentlich bevorzugt 0 Gew.-% beträgt, 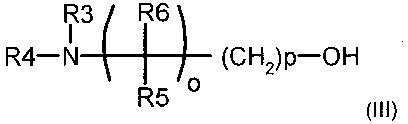 wobei
die Reste R4 und R3 unabhängig voneinander für ein Wasserstoffatom oder eine C₁-C₆-Hydroxyalkylgruppe stehen,
o für die Zahl 0 oder 1 steht,
p für eine ganze Zahl von 1 bis 6 steht, und
die Reste R5 und R6 unabhängig voneinander für eine C₁-C₆-Alkylgruppe stehen.

13. Mittel nach einem der Ansprüche 1 bis 12, **dadurch gekennzeichnet, dass** es zusätzlich mindestens einen ethoxylierten Fettalkohol mit einem Ethoxylierungsgrad von 25 bis 40 enthält, welcher der Formel (IV) entspricht 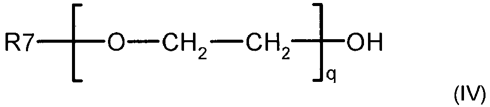 und worin
R7 für eine gesättigte oder ungesättigte, unverzweigte oder verzweigte C₈-C₂₈-Alkylgruppe, bevorzugt für eine gesättigte, unverzweigte C₁₆- oder C₁₈- Alkylgruppe, steht und
q für eine ganze Zahl von 25 bis 40, bevorzugt für eine ganze Zahl von 26 bis 38, weiter bevorzugt für eine ganze Zahl von 27 bis 36 und insbesondere bevorzugt für eine ganze Zahl von 28 bis 34 steht.

14. Verwendung der Kombination aus
(a) mindestens einem ersten Alkylglucosid der Formel (I) 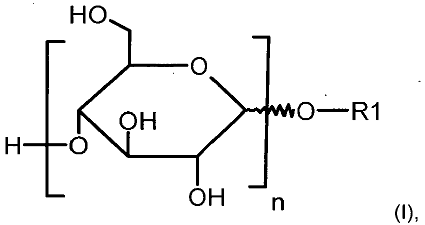 worin R1 für eine unverzweigte oder verzweigte, gesättigte oder ungesättigte C₂₀-C₂₈-Alkylgruppe steht und
n für eine ganze Zahl von 1 bis 10 steht und
(b) mindestens einem zweiten Alkylglucosid der Formel (II) 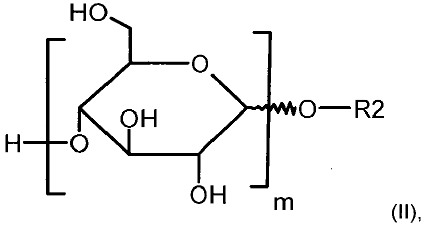 worin R2 für eine unverzweigte oder verzweigte, gesättigte oder ungesättigte C₈-C₁₈-Alkylgruppe steht und
m für eine ganze Zahl von 1 bis 10 steht, und
(c) gegebenenfalls mindestens einem Fettalkohol aus der Gruppe Arachylalkohol (Eicosan-1-ol), Gadoleylalkohol ((9Z*)*-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol), Brassidylalkohol ((13*E*)-Docosen-1-ol), Laurylalkohol (Dodecan-1-ol), Myristylalkohol (Tetradecan-1-ol), Cetylalkohol (Hexadecan-1-ol) und Stearylalkohol (Octadecan-1-ol)
zur Reduktion des Ammoniakgeruches in Mitteln zum oxidativen Färben und/oder oxidativen Aufhellen von keratinischen Fasern, insbesondere menschlichen Haaren, wobei die Mittel in einem kosmetischen Träger - bezogen auf das Gesamtgewicht des anwendungsbereiten Mittels -
(a) ein oder mehrere Alkylglucoside der Formel (I) in einer Gesamtmenge von 0,9 bis 1,5 Gew.-% und
(b) ein oder mehrere Alkylglucoside der Formel (II) in einer Gesamtmenge von 1,2 bis 8,0 Gew.-% und
(c) 0,3 bis 5,5 Gew.-% Ammoniak und
(d) mindestens ein Oxidationsmittel
enthalten.

## Claims

1. An agent for oxidatively dyeing and/or lightening keratin fibers, in particular human hair, containing, in a cosmetic carrier, based on the total weight of the ready-to-apply agent:
(a) one or more alkyl glucosides of formula (I) in a total amount of from 0.9 to 1.5 wt.% HCL 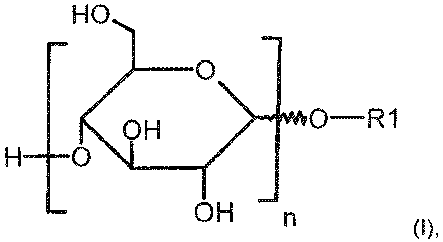 in which R1 represents an unbranched or branched, saturated or unsaturated C₂₀-C₂₈ alkyl group, and
n represents an integer from 1 to 10,
(b) one or more alkyl glucosides of formula (II) in a total amount of from 1.2 to 8.0 wt.%, 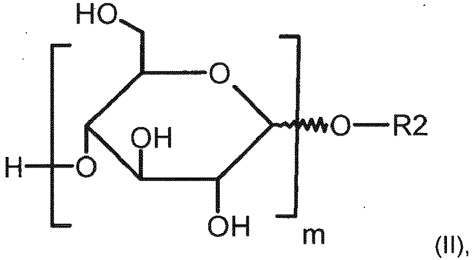 in which R2 represents an unbranched or branched, saturated or unsaturated C₈-C₁₈ alkyl group, and
m represents an integer from 1 to 10,
(c) 0.3 to 5.5 wt.% ammonia, and
(d) at least one oxidizing agent.

2. The agent according to claim 1, **characterized in that** it contains one or more alkyl glucosides (b) of formula (II) in a total amount of from 1.6 to 6.5 wt.%, preferably 2.0 to 5.0 wt.%, and particularly preferably 2.4 to 3.5 wt.%, based on the total weight of the ready-to-apply agent.

3. The agent according to one of claims 1 to 2, **characterized in that** the quantitative ratio of all alkyl glucosides of formula (I) contained in the ready-to-apply agent to all alkyl glucosides of formula (II) contained in the ready-to-apply agent is from 1:2 to 1:10, preferably 1:2 to 1:5.

4. The agent according to one of claims 1 to 3, **characterized in that** it contains ammonia (c) in an amount of from 0.4 to 4.5 wt.%, preferably 0.5 to 3.5 wt.% and particularly preferably 0.6 to 1.1 wt.%, based on the total weight of the ready-to-apply agent.

5. The agent according to one of claims 1 to 4, **characterized in that** it contains, as the oxidizing agent (d), hydrogen peroxide in an amount of from 0.5 to 6.5 wt.%, preferably 1.3 to 5.5 wt.%, more preferably 2.2 to 5.0 wt.% and particularly preferably 3.5 to 4.7 wt.%, based on the total weight of the ready-to-apply agent.

6. The agent according to one of claims 1 to 5, **characterized in that** it additionally contains one or more fatty alcohols from the group consisting of arachidyl alcohol (eicosan-1-ol), gadoleyl alcohol ((9Z)-eicos-9-en-1-ol), arachidonyl alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol), and brassidyl alcohol ((13*E*)-docosen-1-ol) in a total amount of from 0.5 to 7.5 wt.%, preferably 0.7 to 5.0 wt.%, more preferably 0.8 to 4.3 wt.% and particularly preferably 0.9 to 2.6 wt.%, based on the total weight of the ready-to-apply agent.

7. The agent according to one of claims 1 to 6, **characterized in that** it additionally contains one or more fatty alcohols from the group consisting of lauryl alcohol (dodecan-1-ol), myristyl alcohol (tetradecan-1-ol), cetyl alcohol (hexadecan-1-ol) and stearyl alcohol (octadecan-1-ol) in a total amount of from 0.4 to 7.5 wt.%, preferably 0.8 to 6.6 wt.%, more preferably 1.2 to 4.6 wt.% and particularly preferably 1.6 to 2.6 wt.%, based on the total weight of the ready-to-apply agent.

8. The agent according to one of claims 1 to 7, **characterized in that** it additionally contains at least one developer-type oxidation dye precursor and optionally additionally at least one coupler-type oxidation dye precursor.

9. The agent according to one of claims 1 to 8, **characterized in that** it contains, as the first alkyl glucoside (a), at least one compound of formula (I), in which
R1 represents an unbranched, saturated C₂₀ alkyl group and n represents the number 1.

10. The agent according to one of claims 1 to 9, **characterized in that** it contains, as the second alkyl glucoside (b), at least one compound of formula (II), in which R2 represents an unbranched, saturated C₁₆ alkyl group or an unbranched, saturated C₁₈ alkyl group and m represents the number 1.

11. The agent according to one of claims 1 to 10, **characterized in that** the total content of all fatty alcohols contained in the ready-to-apply agent from the group consisting of arachidyl alcohol (eicosan-1-ol), gadoleyl alcohol ((9Z)-eicos-9-en-1-ol), arachidonic alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol), brassidyl alcohol ((13*E*)-docosen-1-ol), lauryl alcohol (dodecan-1-ol), myristyl alcohol (tetradecan-1-ol), cetyl alcohol (hexadecan-1-ol) and stearyl alcohol (octadecan-1-ol), based on the total weight of the ready-to-apply agent, is below 8.0 wt%, preferably below 7.0 wt%, more preferably below 6.0 wt%, and particularly preferably below 5.6 wt%.

12. The agent according to one of claims 1 to 11, **characterized in that** the total amount of all alkanoamines of formula (III) contained in the ready-to-apply agent, based on the total weight of the ready-to-apply agent, is less than 0.1 wt.%, preferably less than 0.05 wt.%, more preferably less than 0.01 wt.% and extremely preferably 0 wt.%, 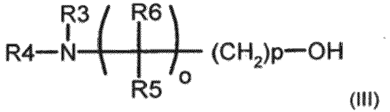 where
the functional groups R4 and R3 represent, independently of one another, a hydrogen atom or a C₁-C₆ hydroxyalkyl group,
o represents the number 0 or 1,
p represents an integer from 1 to 6 and
the functional groups R5 and R6 represent, independently of one another, a C₁-C₆ alkyl group.

13. The agent according to one of claims 1 to 12, **characterized in that** it additionally contains at least one ethoxylated fatty alcohol having a degree of ethoxylation of from 25 to 40, which corresponds to formula (IV) 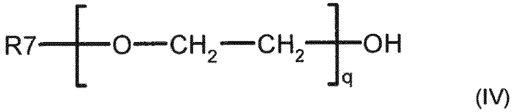 and in which
R7 represents a saturated or unsaturated, unbranched or branched C₈-C₂₈ alkyl group, preferably a saturated, unbranched C₁₆ or C₁₈ alkyl group, and
q represents an integer from 25 to 40, preferably an integer from 26 to 38, more preferably an integer from 27 to 36, and particularly preferably an integer from 28 to 34.

14. The use of the combination of
(a) at least a first alkyl glucoside of formula (I), 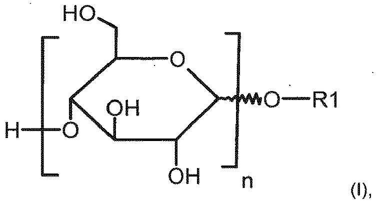 in which R1 represents an unbranched or branched, saturated or unsaturated C₂₀-C₂₈ alkyl group, and
n represents an integer from 1 to 10, and
(b) at least a second alkyl glucoside of formula (II) 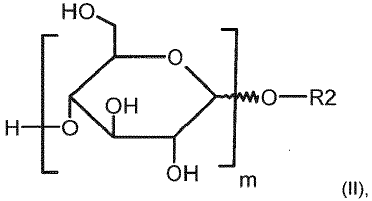 in which R2 represents an unbranched or branched, saturated or unsaturated C₈-C₁₈ alkyl group, and
m represents an integer from 1 to 10, and
(c) optionally at least one fatty alcohol from the group consisting of arachidyl alcohol (eicosan-1-ol), gadoleyl alcohol ((9Z)-eicos-9-en-1-ol), arachidonic alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol), brassidyl alcohol ((13*E*)-docosen-1-ol), lauryl alcohol (dodecan-1-ol), myristyl alcohol (tetradecan-1-ol), cetyl alcohol (hexadecan-1-ol) and stearyl alcohol (octadecan-1-ol)
for reducing the ammonia odor in agents for oxidatively dyeing and/or oxidatively lightening keratin fibers, in particular human hair, wherein the agents contain, in a cosmetic carrier, based on the total weight of the ready-to-apply agent:
(a) one or more alkyl glucosides of formula (I) in a total amount of from 0.9 to 1.5 wt.%, and
(b) one or more alkyl glucosides of formula (II) in a total amount of from 1.2 to 8.0 wt.%, and
(c) 0.3 to 5.5 wt.% ammonia, and
(d) at least one oxidizing agent.

## Revendications

1. Agent de coloration par oxydation et/ou d'éclaircissement de fibres kératiniques, en particulier de cheveux humains, contenant dans un support cosmétique - rapporté au poids total de l'agent prêt à l'emploi -
(a) un ou plusieurs alkylglucosides de la formule (I) dans une quantité totale allant de 0,9 à 1,5 % en poids 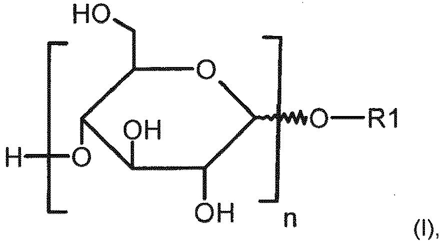 dans laquelle R1 représente un groupe alkyle en C₂₀-C₂₈ non ramifié ou ramifié, saturé ou insaturé, et
n représente un nombre entier allant de 1 à 10,
(b) un ou plusieurs alkylglucosides de la formule (II) dans une quantité totale allant de 1,2 à 8,0 % en poids, 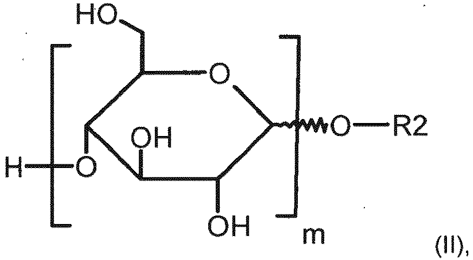 dans laquelle R2 représente un groupe alkyle en C₈-C₁₈ non ramifié ou ramifié, saturé ou insaturé, et
m représente un nombre entier allant de 1 à 10,
(c) 0,3 à 5,5 % en poids d'ammoniac et
(d) au moins un agent oxydant.

2. Agent selon la revendication 1, **caractérisé en ce qu'**il contient un ou plusieurs alkylglucosides (b) de la formule (II) dans une quantité totale allant de 1,6 à 6,5 % en poids, de manière préférée de 2,0 à 5,0 % en poids et de manière particulièrement préférée de 2,4 à 3,5 % en poids - rapporté au poids total de l'agent prêt à l'emploi.

3. Agent selon l'une des revendications 1 à 2, **caractérisé en ce que** le rapport quantitatif de tous les alkylglucosides de la formule (I) contenus dans l'agent prêt à l'emploi sur tous les alkylglucosides de la formule (II) contenus dans l'agent prêt à l'emploi s'établit de 1:2 à 1:10, de manière préférée de 1:2 à 1:5.

4. Agent selon l'une des revendications 1 à 3, **caractérisé en ce qu'**il contient de l'ammoniac (c) dans une quantité allant de 0,4 à 4,5 % en poids, de manière préférée de 0,5 à 3,5 % en poids et de manière particulièrement préférée de 0,6 à 1,1 % en poids - rapporté au poids total de l'agent prêt à l'emploi.

5. Agent selon l'une des revendications 1 à 4, **caractérisé en ce qu'**il contient, en tant qu'agent d'oxydation (d), du peroxyde d'hydrogène dans une quantité allant de 0,5 à 6,5 % en poids, de manière préférée de 1,3 à 5,5 % en poids, de manière davantage préférée de 2,2 à 5,0 % en poids et de manière particulièrement préférée de 3,5 à 4,7 % en poids - rapporté au poids total de l'agent prêt à l'emploi.

6. Agent selon l'une des revendications 1 à 5, **caractérisé en ce qu'**il contient en outre un ou plusieurs alcools gras choisis parmi le groupe constitué par l'alcool arachylique (éicosan-1-ol), l'alcool gadoléylique ((9Z)-éicos-9-ène-1-ol), l'alcool arachidonique ((5Z,8Z,11Z,14Z)-éicosa-5,8,11,14-tétraène-1-ol), l'alcool hénéicosylique (hénéicosane-1-ol), l'alcool béhénylique (docosane-1-ol), l'alcool érucylique ((13Z)-docos-13-ène-1-ol) et l'alcool brassidylique ((13E)-docosène-1-ol) dans une quantité totale allant de 0,5 à 7,5 % en poids, de manière préférée de 0,7 à 5,0 % en poids, de manière davantage préférée de 0,8 à 4,3 % en poids et de manière particulièrement préférée de 0,9 à 2,6 % en poids - rapporté au poids total de l'agent prêt à l'emploi.

7. Agent selon l'une des revendications 1 à 6, **caractérisé en ce qu'**il contient en outre un ou plusieurs alcools gras choisis parmi le groupe constitué par l'alcool laurylique (dodécane-1-ol), l'alcool myristylique (tétradécane-1-ol), l'alcool cétylique (hexadécane-1-ol) et l'alcool stéarylique (octadécane-1-ol) dans une quantité totale allant de 0,4 à 7,5 % en poids, de manière préférée de 0,8 à 6,6 % en poids, de manière davantage préférée de 1,2 à 4,6 % en poids et de manière particulièrement préférée de 1,6 à 2,6 % en poids - rapporté au poids total de l'agent prêt à l'emploi.

8. Agent selon l'une des revendications 1 à 7, **caractérisé en ce qu'**il contient de plus au moins un précurseur de colorant par oxydation de type développeur, et le cas échéant contient de plus au moins un précurseur de colorant par oxydation de type coupleur.

9. Agent selon l'une des revendications 1 à 8, **caractérisé en ce qu'**il contient, en tant que premier alkylglucoside (a), au moins un composé de la formule (I), dans laquelle R1 représente un groupe alkyle en C₂₀ non ramifié, saturé, et n représente le nombre 1.

10. Agent selon l'une des revendications 1 à 9, **caractérisé en ce qu'**il contient, en tant que second alkylglucoside, (b) au moins un composé de la formule (II), dans laquelle R2 représente un groupe alkyle en C₁₆ non ramifié, saturé, ou un groupe alkyle en C₁₈ non ramifié, saturé, et m représente le nombre 1.

11. Agent selon l'une des revendications 1 à 10, **caractérisé en ce que** la teneur totale de tous les alcools gras contenus dans l'agent prêt à l'emploi, choisis parmi le groupe constitué par l'alcool arachylique (éicosan-1-ol), l'alcool gadoléylique ((9Z)-éicos-9-ène-1-ol), l'alcool arachidonique ((5Z,8Z,11Z,14Z)-éicosa-5,8,11,14-tétraène-1-ol), l'alcool hénéicosylique (hénéicosane-1-ol), l'alcool béhénylique (docosane-1-ol), l'alcool érucylique ((13Z)-docos-13-ène-1-ol) et l'alcool brassidylique ((13E)-docosène-1-ol), l'alcool laurylique (dodécane-1-ol), l'alcool myristylique (tétradécane-1-ol), l'alcool cétylique (hexadécane-1-ol) et l'alcool stéarylique (octadécane-1-ol) - rapporté au poids total de l'agent prêt à l'emploi - est inférieure à 8,0 % en poids, de manière préférée est inférieure à 7,0 % en poids, de manière davantage préférée est inférieure à 6,0 % en poids et de manière particulièrement préférée est inférieure à 5,6 % en poids.

12. Agent selon l'une des revendications 1 à 11, **caractérisé en ce que** la quantité totale de toutes les alcanolamines de formule (III) contenues dans l'agent prêt à l'emploi est - rapportée au poids total de l'agent prêt à l'emploi - inférieure à 0,1 % en poids, de préférence inférieure à 0,05 % en poids, de manière davantage préférée inférieure à 0,01 % en poids et de manière préférée entre toutes est de 0 % en poids, 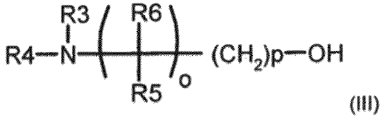 dans laquelle
les radicaux R4 et R3 représentent indépendamment l'un de l'autre un atome d'hydrogène ou un groupe hydroxyalkyle en C₁-C₆,
o représente le nombre 0 ou 1,
p représente un nombre entier allant de 1 à 6, et
les radicaux R5 et R6 représentent indépendamment l'un de l'autre un groupe alkyle en C₁-C₆.

13. Agent selon l'une des revendications 1 à 12, **caractérisé en ce qu'**il contient de plus au moins un alcool gras éthoxylé avec un degré d'éthoxylation allant de 25 à 40, lequel correspond à la formule (IV) 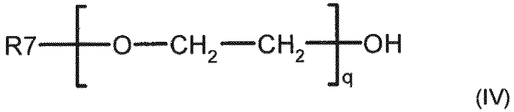 et dans laquelle
R7 représente un groupe alkyle en C₈-C₂₈ saturé ou insaturé, non ramifié ou ramifié, de manière préférée un groupe alkyle en C₁₆ ou C₁₈ saturé, non ramifié, et
q est un nombre entier allant de 25 à 40, de manière préférée un nombre entier allant de 26 à 38, de manière davantage préférée un nombre entier allant de 27 à 36, et de manière particulièrement préférée un nombre entier allant de 28 à 34.

14. Utilisation de la combinaison constituée de
(a) au moins un premier alkylglucoside de la formule (I) 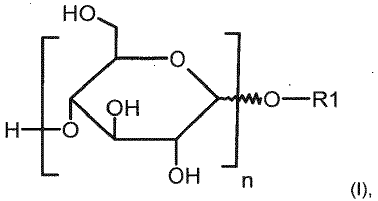 dans laquelle R1 représente un groupe alkyle en C₂₀-C₂₈ non ramifié ou ramifié, saturé ou insaturé, et
n représente un nombre entier allant de 1 à 10, et
(b) au moins un second alkylglucoside de la formule (II) 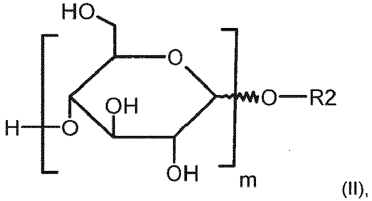 dans laquelle R2 représente un groupe alkyle en C₈-C₁₈ non ramifié ou ramifié, saturé ou insaturé, et
m représente un nombre entier allant de 1 à 10, et
(c) le cas échéant au moins un alcool gras choisi parmi le groupe constitué par l'alcool arachylique (éicosan-1-ol), l'alcool gadoléylique ((9Z)-éicos-9-ène-1-ol), l'alcool arachidonique ((5Z,8Z,11Z,14Z)-éicosa-5,8,11,14-tétraène-1-ol), l'alcool hénéicosylique (hénéicosane-1-ol), l'alcool béhénylique (docosane-1-ol), l'alcool érucylique ((13Z)-docos-13-ène-1-ol) et l'alcool brassidylique ((13E)-docosène-1-ol), l'alcool laurylique (dodécane-1-ol), l'alcool myristylique (tétradécane-1-ol), l'alcool cétylique (hexadécane-1-ol) et l'alcool stéarylique (octadécane-1-ol)
destiné à réduire l'odeur de l'ammoniac dans des agents de coloration par oxydation et/ou d'éclaircissement par oxydation de fibres kératiniques, en particulier de cheveux humains, dans laquelle les agents contiennent dans un support cosmétique - rapporté au poids total de l'agent prêt à l'emploi -
(a) un ou plusieurs alkylglucosides de la formule (I) dans une quantité totale allant de 0,9 à 1,5 % en poids et
(b) un ou plusieurs alkylglucosides de la formule (II) dans une quantité totale allant de 1,2 à 8,0 % en poids et
(c) 0,3 à 5,5 % en poids d'ammoniac et
(d) au moins un agent oxydant.
